# EUROPEAN PATENT APPLICATION

(11) **EP 2 295 100 A2**
(43) Date of publication of application: **16.03.2011**
(21) Application number: 10015561.3
(22) Date of filing: 24.10.2007
(51) Int. Cl.: A61M 5/36, B65B 3/00, B65B 57/14, G01N 21/90, G01N 21/49

(54) **Optical inclusion sensor**

(30) Priority: 24.10.2006 US 853942 P
(62) Divisional of application: 07839782.5
(71) Applicant: MALLINCKRODT, INC., Hazelwood, MO 63042 (US)
(72) Inventor: Bruce, John K., Burlington Kentucky 41005 (US); Gibbs, Jonathan D., Mason Ohio 45040 (US)
(74) Representative: Chettle, Adrian John

(57) **Abstract**

A sensor for sensing inclusions in a containerized medical fluid. In certain embodiments, the sensor may include an image capture device and an image processor communicatively interconnected to the image capture device. The image processor may include an inclusion identifier configured to detect data in an image indicative of inclusions in the medical fluid.

## Description

### FIELD OF THE INVENTION

The invention relates generally to quality control for filled syringes, and, more specifically, to an apparatus and method for capturing and evaluating images of syringes to identify the presence of bubbles or other foreign material in the syringes.

### BACKGROUND

This section is intended to introduce the reader to various aspects of art that may be related to various aspects of the present invention, which are described and/or claimed below. This discussion is believed to be helpful in providing the reader with background information to facilitate a better understanding of the various aspects of the present invention. Accordingly, it should be understood that these statements are to be read in this light, and not as admissions of prior art.

Syringes are typically filled with a liquid prior to their use. Generally, a health care professional or manufacturer of pre-filled syringes fills the syringe with a drug, contrast agent, or other medically useful liquid. After filling, but before injection, the syringe may be inspected for the presence of materials other than the liquid to be injected, such as bubbles or foreign particles (collectively referred to herein as "inclusions"). Typically, a human inspector visually examines the syringe and attempts to quantify the amount of these materials. A decision whether to use the syringe is typically made based on the visual inspection. Unfortunately, visual inspection is often labor intensive, and the resulting measurement frequently varies due to the subjective judgment of individual inspectors.

### SUMMARY

Certain aspects of the present invention are set forth below. It should be understood that these aspects are presented merely to provide the reader with a brief summary of certain forms the invention might take and that these aspects are not intended to limit the scope of the invention. Indeed, the invention may encompass a variety of aspects that may not be set forth below.

In certain aspects, the present invention relates to a system for objectively detecting and/or quantifying inclusions in a medical fluid. Some of the subsequently discussed embodiments digitally image the contents of a filled medical fluid container to quantify a number of inclusions (i.e., undesired substances such as bubbles and/or particles) in the medical fluid. In certain embodiments, this quantifying entails examining one or more pixels in an image of the medical fluid to determine whether the pixel(s) depict(s) a portion of an inclusion and/or the fluid. In some of these embodiments, pixels having a color and/or intensity within a certain range may be classified as depicting an inclusion. If the number or percentage of pixels classified as depicting an inclusion is above a threshold number, the syringe may be determined to be unusable in some embodiments of the present invention.

A first aspect of the present invention is directed to an inclusion sensor for sensing inclusions in a containerized medical fluid. The sensor includes an image capture device (e.g., a digital camera) and an image processor communicatively interconnected with the image capture device. Incidentally, "communicatively interconnected with" or the like generally refers to a condition in which a plurality of things are coupled (either directly or indirectly) in a manner that enables signal, such as electrical and/or radio wave signal, to be communicated therebetween. In addition to the image processor and the image capture device, the sensor also includes an inclusion identifier that is communicatively interconnected with the image processor. This inclusion identifier is configured to identify data in an image of a medical fluid that is indicative of inclusions in the medical fluid.

A second aspect of the invention is directed to an inclusion meter for quantifying inclusions in a medical fluid. The meter includes an image capture device and an inclusion counter communicatively interconnected with the image capture device. The inclusion counter is configured to process (e.g., quantify) image data indicative of inclusions in the medical fluid.

Yet a third aspect of the invention is directed to a method of detecting inclusions in a medical fluid. In this method, a medical fluid is imaged, and a statistic based on image data representative of inclusions in the medical fluid is calculated. The statistic may be stored to memory, displayed (e.g., on a computer screen), and/or utilized to trigger output of a signal (e.g., visual and/or audible alarm) indicating that the statistic exceeds an acceptable inclusion tolerance level.

Still a fourth aspect of the invention is directed to a method of operation for a medical fluid injector system. In this method, a syringe containing a medical fluid is received by (e.g., mounted on or to) the medical fluid injector system. A signal indicative of a quantity of image data indicative of inclusions in the medical fluid is then generated by the system. The system then makes a determination of whether the signal exceeds a predetermined threshold value.

Still yet a fifth aspect of the invention is directed to a tangible medium having code stored thereon for use in detecting the presence of inclusions within a medical fluid. In some embodiments, the code includes instructions for receiving data representative of an image of medical fluid, analyzing the data, and creating an indication of a quantity of inclusions within the medical fluid based on the analysis.

Various refinements exist of the features noted above in relation to the various aspects of the present invention. Further features may also be incorporated in these various aspects as well. These refinements and additional features may exist individually or in any combination. For instance, various features discussed below in relation to one or more of the illustrated embodiments may be incorporated into any of the above-described aspects of the present invention alone or in any combination. Again, the brief summary presented above is intended only to familiarize the reader with certain aspects and contexts of the present invention without limitation to the claimed subject matter.

### BRIEF DESCRIPTION OF THE FIGURES

These and other features, aspects, and advantages of the present invention will become better understood when the following detailed description is read with reference to the accompanying figures in which like characters represent like parts throughout the figures, wherein:

FIG. 1 is a diagram of an exemplary bubble detector;

FIG. 2 is a perspective view of another exemplary bubble detector;

FIG. 3 is a flow chart depicting an exemplary injection process;

FIGS. 4 and 5 are exemplary images of a fluid with and without an acceptable quantity of bubbles, respectively; and

FIG. 6 is a perspective view of an exemplary integrated bubble detector.

### DETAILED DESCRIPTION OF SPECIFIC EMBODIMENTS

One or more specific embodiments of the present invention will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a," "an," "the," and "said" are intended to mean that there are one or more of the elements. The terms "comprising," "including," and "having" are intended to be inclusive and mean that there may be additional elements other than the listed elements. Moreover, the use of "top," "bottom," "above," "below" and variations of these terms is made for convenience, and does not require any particular orientation of the components. The term "disposed about," as in "a first object is disposed about another," is intended to indicate that the first object at least substantially, though not necessarily completely, envelops or surrounds the other object. Further, as used herein, the term "inclusion" refers to material within a fluid (e.g., a liquid) that is optically distinct from the fluid and/or measurably optically affects the fluid, e.g., bubbles, solid particulates, a precipitate, a portion of the fluid that has undergone a chemical reaction, or another fluid that induces a color change in the fluid. Herein, the term "containerized" generally means packaged. The term "coupled" refers to the state of being directly or indirectly, permanently or removeably, mechanically connected or in contact, and "communicatively interconnected" refers to a condition that enables information to be conveyed between a first object and a second object (e.g., through hardwire connection, wireless signal transmission, etc.). As used herein, the term "affixed" refers to the condition of being held at least temporarily in fixed relation (e.g., with zero degrees of relative freedom).

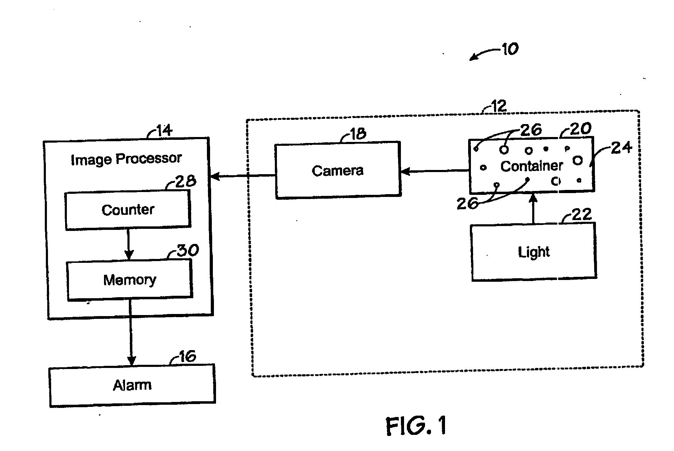

Turning to the figures, FIG. 1 illustrates an exemplary inclusion detector 10. As explained further below, the presently discussed inclusion detector 10 may illuminate a filled syringe, capture an image of the fluid therein, and quantify an amount of bubbles and/or other inclusions within the syringe by analyzing the image. The exemplary inclusion detector 10 may, in certain embodiments, reduce the subjectivity and cost associated with manual quality control inspection of syringes.

The exemplary inclusion detector 10 includes a sensor assembly 12, an image processor 14, and an alarm 16. As used herein, the term "image processor" refers to any device that accepts as an input an image (the definition of which herein also includes data representative of an image) and provides (e.g., calculates) an output that is at least in part determined by the input. These components 12,14,16 are described in much more detail below. For now, it is sufficient to note that the sensor assembly 12 is communicatively interconnected with the image processor 14, and the image processor 14 is communicatively interconnect with the alarm 16. In operation, the sensor assembly 12 captures at least one image of a medical fluid, and the image processor 14 may trigger the alarm 16 based on an analysis of the captured image. To this end, in certain embodiments, the exemplary image sensor assembly 12, image processor 14, and alarm 16 may include a variety of sub-components that are discussed in greater detail below.

The sensor assembly 12 of FIG. 1 includes a camera 18, a container 20, and a light 22. In some embodiments, the camera 18 may include or refer to a digital camera, a webcam, an image capture board, a light sensor array, a two-dimensional light sensor array, a video camera, or another appropriate image capture device. The camera 18 may have a light sensitive medium, such as film, a charge coupled device (CCD), a complementary metal-oxide semiconductor sensor, or an array of photo-diodes, for instance. The resolution of the camera 18 may be greater than or equal to one-hundred pixels, one-thousand pixels, one-hundred-thousand pixels, one-million pixels, two-million pixels, three-million pixels, four-million pixels, five-million pixels, ten-million pixels, or more, for example. The camera 18 may be sensitive to visible light, infra-red light, and/or ultra-violet light, depending on factors such as the desired application.

The container 20 may be a syringe, an intravenous fluid bag, bulk fluid storage, an injecting device, or a fluid conduit between a patient and a syringe. For example, the container 20 may be a pre-filled syringe. Walls of the container 20 may include a generally transparent or generally translucent portion through which light may pass. In some embodiments, substantially all of the walls of the container 20 are made of a generally transparent material, such as glass or another appropriate medical fluid container material (e.g., Daikyo Resin CZ available from West Pharmaceutical Services of Lionville, Pennsylvania).

The container 20 may contain a fluid 24 and a number of inclusions 26 within or adjacent the fluid 24. In some embodiments, the fluid 24 may be saline, a tagging agent, a contrast agent, a radiopharmaceutical or other appropriate medical fluid. The inclusions 26 may be gas bubbles and/or solid particles for instance, It should be noted that some embodiments may not include a fluid 24 or inclusions 26, which is not to suggest that other features discussed herein may not also be omitted. For example, some embodiments may include a fluid that is not necessarily a liquid, such as a gas or a gel, or they may simply include a container 20 and inclusions 26 within an otherwise empty container 20.

The sensor assembly 12 includes a light 22. The light 22 may have or refer to one or more types of lights, such as a light emitting diode, an organic light emitting diode, a fluorescent light, an incandescent light, natural light, and/or ambient lighting in a room housing the sensor assembly 12, for example. The light 22 may include a single light or an array of lights. The light 22 may emit light at a variety of wavelengths, depending on the embodiment. For instance, the light 22 may emit infrared light, visible light, ultraviolet light, or a combination thereof. In some embodiments, the light 22 may emit light at multiple wavelengths, such as generally white light.

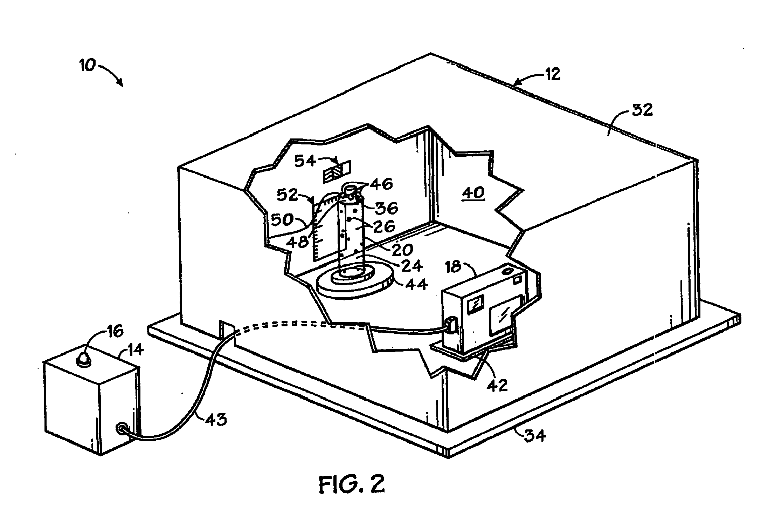

As assembled, the light 22 and the camera 18 are positioned to illuminate and image (respectively) the container 20. In certain embodiments, the light 22 may have an area of illumination, which may be defined as an area that the light 22 illuminates when energized. The light 22 may be positioned on the container 20 or a light fixture so that emitted light shines on or through the container 20. The camera 18 has a field of view, which may be defined as the area depicted by an image captured by the camera 18. The camera 18 may be coupled to a camera fixture and oriented facing the container 20, and the container 20 may be coupled to a container fixture (as explained further in reference to FIG. 2 below).

To potentially enhance repeatability of measurements, in certain embodiments, the camera 18, container 20, and light 22 may be aligned to mounts (examples of which are discussed below) when an image is captured. As used herein, the term "mount" generally refers to any device that aligns an object in a position and/or orientation, and the phrase "aligned to" refers to the condition of being positioned and/or oriented with respect to some reference structure, such as a mount. In some embodiments, the camera 18, container 20, and/or light 22 may be removably or permanently coupled to the sensor assembly 12 by mounts at one or more discrete locations in one or more discrete orientations so that multiple measurements may be performed under substantially similar or nearly identical conditions. To possibly further enhance repeatability, the sensor assembly 12 of some embodiments may obstruct light from sources other than the light 22.

Turning to other components of the inclusion detector 10 of FIG. 1, the image processor 14 may include a counter 28 and a memory 30. The counter 28 may include code and/or circuits configured to output a signal indicative of a quantity of inclusions 26 in the fluid 24. Alternatively or additionally, the image processor 14 may include an inclusion identifier configured to indicate or detect the presence of inclusions 26 appearing in an image. The counter 28 may include a microprocessor, digital signal processor, central processing unit, solid state switch, and/or an application specific integrated circuit. In some embodiments, a personal computer, mainframe computer, server, personal digital assistant, handheld computer, tablet computer, and/or laptop may embody all or part of the counter 28. Alternatively or additionally, the counter 28 may include an analog integrator circuit including a capacitor coupled to a voltmeter. While the counter 28 is represented as a monolithic block in the present embodiment, it should be noted that the counter 28 may include a variety of devices that are physically located remote from one another and, thus, should be regarded as representing a logical unit, e.g., a state machine.

The memory 30 may include various forms of memory, such as dynamic random access memory, flash memory, a hard disc drive, a tape drive, an optical drive, static random access memory, and/or other tangible machine readable mediums, for example. In some embodiments, the memory 30 may be integrated into the counter 28 (e.g., as cache memory), or the memory 30 may be remote from the counter 28 (e.g., in a database on a remote server). As with the counter 28, the memory 30 is intended to represent a logical unit and not necessarily a single integrated device, though such devices may be employed in some embodiments. It should be noted that, as with many of the features discussed herein, some embodiments may not include memory 30.

The memory 30 may store programs that facilitate operation of the counter 28, such as an operating system, image analysis software, spreadsheet software, statistical process control software, networking software, and/or database software, for instance. In some embodiments, the memory 30 may store code for receiving an image of the medical fluid 24, analyzing the image, and outputting an indication of a proportion or quantity of inclusions 26 appearing within the image. In some embodiments, the code stored in the memory 30 may include instructions for analyzing data in the image to determine if pixels are darker or lighter than a threshold value and for calculating the percentage of the image that depicts an inclusion 26.

As assembled, the counter 28 is communicatively interconnected with the camera 18 and the memory 30. The counter 28 may communicate with the camera 18 via an image input and an image capture application programming interface (API), such as version 2.0 of the TWAIN (technology without an interesting name) standard published by the TWAIN working group. The counter 28 may be coupled to the memory 30 via a memory bus, a northbridge of a mother-board, a memory controller, and/or other device suitable for transmitting data. The counter 28 and/or memory 30 also may be communicatively interconnected with the alarm 16.

The alarm 16 may have an audio, visual, and/or mechanical indicator that signals the quantity, percentage, and/or presence of inclusions 26 within a container 20. For instance, the alarm 10 may include a speaker, a light, and/or monitor. Alternatively or additionally, the alarm 16 may include a mechanical device to physically interact with a container 20 in a manner indicative of the quantity and/or presence of inclusions 26, such as a factory automation system that places containers 20 in bins based on the quantity/presence of inclusions 26 detected therein, for instance.

The signal from the alarm 16 may be analog or digital. For instance, the alarm 16 may generate a signal indicative of one of two levels of inclusions (i.e., a binary signal), one of three levels of inclusions, one of four levels of inclusions, one of five levels of inclusions, and so on. In another embodiment, the alarm 16 may display digits indicative of the level of inclusions. Alternatively, the signal from the alarm 16 may be an analog signal indicative of the quantity of inclusions, such as the intensity or wavelength of a light or a sound. It should be noted that some embodiments may not include an alarm 16, which is not to suggest that other features discussed herein may not also be omitted.

FIG. 2 is a cut-away, perspective view of the inclusion detector 10 that depicts more details of the sensor assembly 12. In the present embodiment, the sensor assembly 12 includes a light shield 32, a chassis 34, and a light fixture 36. The light shield 32 may include or refer to a curtain, a box, a room, or other appropriate light shielding that is disposed about the camera 18, the container 20, and the light 22. In some embodiments, the light shield 32 may be formed from a substantially opaque material to limit the amount of ambient light that reaches an interior 40 of the light shield 32. To facilitate access to the interior 40, in certain embodiments, the light shield 32 may be removably coupled (i.e., attached directly or indirectly in a manner that enables removal) to the chassis 34, and/or a portion of the light shield 32 or chassis 34 may include a passageway that may be selectively covered (e.g., a door, access cover, or removable panel).

The chassis 34 includes a camera mount 42 and a container mount 44. In some embodiments, the camera mount 42 may include a camera bracket or a camera alignment mark. Similarly, the container mount 44 may include a syringe mount, a syringe receptacle, a syringe holder, or a container alignment mark. The illustrated camera mount 42 is spaced away from the container mount 44 and oriented so as to point the camera 18 at a container 20 aligned to the container mount 44. A camera cable 43 is utilized in this embodiment to communicatively interconnect the camera 18 with the image processor 14.

The light fixture 36 illustrated in FIG. 2 includes a ring of generally regularly spaced light emitting diodes 46. The light fixture 36 may be removably disposed on a tapered portion or neck 48 of the container 20. In some embodiments, the light fixture 36 may rest on the neck 48. In certain other embodiments, the light fixture 36 may be coupled to other parts of the sensor assembly 12, such as the light shield 32, the chassis 34, or the camera 18. Power cables 50 may deliver power to the diodes 48 from a power supply or the image processor 14, for example.

In some embodiments, a screen, grid, or other array of spatial reference marks 52 may be placed near the container 20 in the field of view of the camera 18. For example, a grid may be placed in front of, along side of, or behind the container 20 to provide reference points used to determine a spatial distribution of inclusions 26 within the fluid 24. Additionally, the image processor 14 or other circuitry may spatially calibrate images of the container 20 with reference to the spatial reference marks 52. For instance, the image processor 14 may determine that the spatial reference marks 52 in the horizontal direction appear closer together than expected and adjust the horizontal distribution of pixels in the image to compensate. Further, the spatial reference marks 52 may facilitate calculating the physical location and distribution of the inclusions 26 within the container 20. In some embodiments, the image processor 14 may determine the spatial distribution of inclusions 26 by the coordinates of pixels depicting the inclusions 26.

Additionally, calibration marks 54 may be placed in view of the camera 18. In some embodiments, the calibration marks 54 include an array of colored marks near the container 20. An image of the container 20 may be adjusted based on an actual color of a calibration mark 54 in the image and an expected or known color of the calibration mark 54. For instance, if a red calibration mark 54 appears orange in the image, the red component of the pixels in all or part of the image may be increased to calibrate the image. Similarly, the white balance, tint, darkness, contrast, alignment, and other properties of the image may be adjusted with reference to calibration marks 54. The image processor 14 or other suitable circuitry may be configured to calibrate the image based on the appearance of calibration marks 54 in the image.

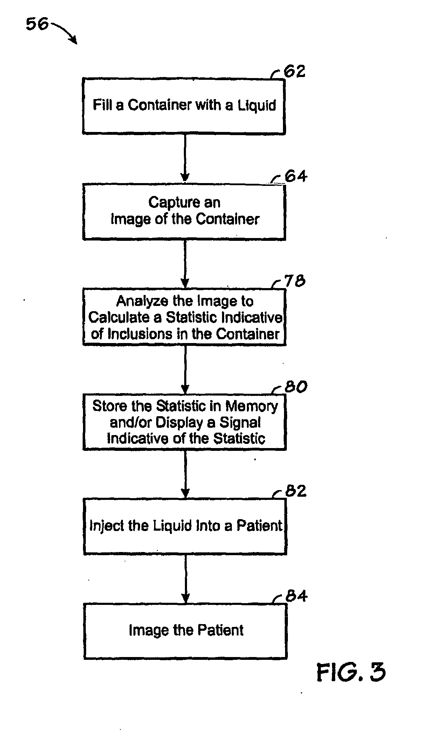

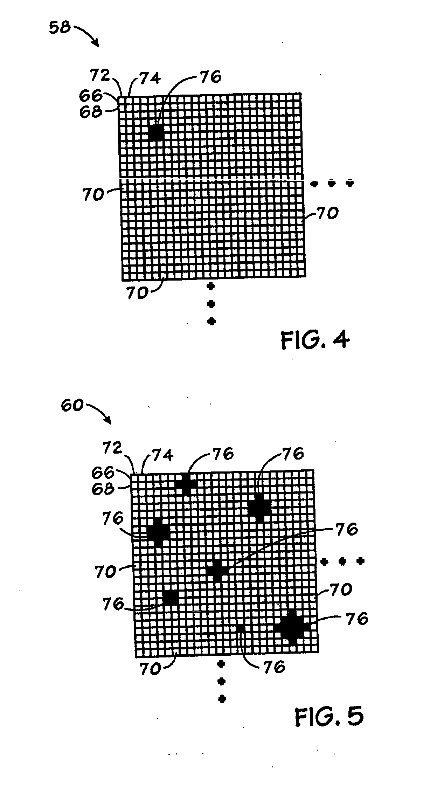

Operation of the inclusion detector 10 will now be described with reference to FIGS. 3-5. FIG. 3 is a flow chart illustrating an exemplary injection process 56, and FIGS. 4, 5 depict portions of digital images 58, 60 of containers 20 having and exceeding an acceptable number of inclusions 26, respectively.

Turning to FIG. 3, the exemplary injection process 56 begins with filling a container 20 with a fluid 24 (here, a liquid), as depicted by block 62. A patient, a nurse, a medical technician, a doctor, or a syringe manufacturer, among others, may fill the container 20.

Next in the present embodiment, an image 58, 60 of the container 20 is captured, as depicted by block 64. The camera 18 depicted in FIGS. 1, 2 may capture the image 58, 60 in response to a signal from the image processor 14, the presence of the container 20 in the container mount 44, or manual activation by an operator, for instance. The camera 18 may be focused on the entire depth of the container 20, a front portion of the container 20, or a back portion of the container 20, for instance. In some embodiments, multiple images 58, 60 under different conditions may be captured. For example, the container 20 may be lit with light of a different intensity, color, orientation, polarization, and/or degree of coherence in consecutive images. In some embodiments, different images 58, 60 may be captured from different sides (e.g., front, back, left, right, etc.), different angles (e.g., 30, 45, 60, and 90 degrees), or with different camera settings. For instance, the camera 18 may be focused to different depths in different images. The different conditions may tend to highlight certain kinds of inclusions 26, such as inclusions 26 located in a particular portion of the container 20 or inclusions 26 of a particular size.

Examples of images 58, 60 that may be acquired during image capture are depicted by FIGS. 4, 5. The images 58, 60 include horizontal rows 66, 68 of pixels 70 and vertical columns 72, 74 of pixels 70. The images 58, 60 may depict all or part of the container 20 and, optionally, an area surrounding the container 20. The pixels 70 may each have a corresponding intensity value or values, for example, a grey-scale value or red-green-blue intensity values, and coordinates, such as a horizontal and vertical coordinates. In the present example, dark portions 76 of the image 58, 60 depict inclusions 26 in the fluid 24. In other embodiments, inclusions 26 may be depicted by bright portions of the image 58, 60.

Next in the exemplary injection process 56, the image processor 14 is utilized to analyze the image 58, 60 to calculate statistics indicative of inclusions 26 within the container 20, as depicted by block 78. The image processor 14 may calibrate the image 58, 60 with reference to the spatial reference marks 52 and/or the calibration marks 54 as part of the analysis. The calibration may enhance the repeatability of results and account for drift in the camera 18 and/or the light 22. To identify inclusions 26, the image processor 14 may analyze each pixel 70 of the image 58, 60, proceeding along each horizontal row 66, 68, in a row by row manner. Alternatively, the image processor 14 may analyze each pixel 70 in a column by column manner. At each pixel 70, the image processor 14 may determine whether the pixel 70 is darker or lighter than a threshold value. Alternatively, the image processor 14 may determine if the pixel 70 depicts a particular range of color, e.g., more or less red than a threshold value, more or less green than a threshold value, or more or less blue than a threshold value. The pixel 70 may be categorized based a grey-scale intensity value, a digital black or white value, a red intensity value, a blue intensity value, a green intensity value, or a combination thereof, for example. For example, pixels darker than a threshold value may be categorized as indicating the presence of an inclusion 26. The threshold value to which pixels 70 are compared may be a local average of other proximate pixels 70, an average value for pixels 70 of the same or similar coordinates across a number of images 58, 60, and/or a value set in advance and stored in memory 30, for instance. In some embodiments, the analysis may be carried out only on a portion of the pixels in a given image. In such embodiments, the selected pixels to be analyzed may be random or may be one or more predetermined groups of pixels, for instance.

A number of statistics indicative of inclusions 26 may be calculated. For example, the percentage or number of pixels 70 that indicate the presence of an inclusion 26 may be calculated. In another example, an edge-detection algorithm or other form of analysis may be employed to calculate the size of one or more inclusions 26, the distribution of sizes of inclusions 26, and/or the number of inclusions 26. Some embodiments may include profiles of known forms of inclusions 26 in memory 30 and match the known forms to dark spots 76 in the images 58, 60.

In some embodiments, additional data about the container 20 may be extracted from the image 58, 60. For instance, the type of container 20 may be identified based on the shape and/or color of the container 20. In some embodiments, the image processor 14 may measure a diameter of the container 20 based on the number of pixels 70 in a horizontal row 66, 68 that have a particular color or intensity. In another example, the image process may calculate a diameter based on the number of pixels 70 between points on a horizontal row 66, 68 where the color or intensity changes by more than a certain amount or rate. Similarly, vertical dimensions of the container 20 may be measured based on the color or intensity of pixels 70 in a vertical column 72, 74. In other words, the image processor 14 may detect the edges of the container 20 in the image 58, 60 and measure a dimension of the container 20 based on the location of the edges in the image 58, 60. The measured dimension may then be matched with a dimension of a known container in memory 30, and the image processor 14 may identify the container 20. In another example, a label on the container 20, such as bar code or radio frequency tag, may be identified and read by the image processor 14.

Information about the fluid 14 may be extracted from the image 58, 60 by the image processor 14. For example, the color of the fluid 24 may be indicated by pixel 70 color intensity values that are included in the image 58, 60. The color of the fluid 24 may indicate the type of fluid 24 and/or various properties of the fluid 24, such as concentration of constituent materials, age, temperature, degree of settling, etc. In some embodiments, dimensions of a meniscus of the fluid 24 may be measured by the image processor 14. Surface tension of the fluid 24 may be calculated by the image processor 14 based on the measured dimensions of the meniscus. As a final example, the amount of fluid 24 within the container 20 may be measured by, for instance, identifying the type of container 20 and the number of pixels 70 between where the top of the container 20 appears in the image 58, 60 and where the top of the fluid 24 appears in the image 58, 60.

In embodiments that capture two images 58, 60 of the same container 20 under different conditions, additional data about the inclusions 26 may be calculated. For example, movement of the inclusions 26 may be detected and measured, inclusions 26 disposed at different focal lengths from the camera 18 may be identified, inclusions 26 viewable under different wavelengths of light may be identified and distinguished, and/or bright-field and dark-field measurements may be compared.

After analyzing the image 58, 60, the image processor 14 may store the resulting statistics in memory 30 and/or display a signal indicative of the statistics, as depicted by block 80 of FIG. 3. The types of signals indicative of the statistic are myriad. For example, the alarm 16 may sound, flash, change state, or light-up. In another example, a value or shape corresponding to the statistic may be displayed on a monitor. In some embodiments, a factory automation system may signal the value of the statistic by placing the container 20 in a particular location, such as a box to be shipped or a bin for rejected containers 20. Alternatively, or additionally, a label may be printed with the statistic or information corresponding to the statistic, and the label may be placed on the container 20 or a box of containers 20. In certain embodiments, the statistic or data corresponding to the statistic may be written to a radio frequency tag affixed to the container 20 or a box of containers 20 by a radio frequency device that may be communicatively interconnected with the image processor 14. For instance, in some embodiments, inclusion data describing whether or not the medical fluid in the container 20 includes more or less inclusions than some predetermined threshold amount may be written to the radio frequency tag on the container 20. Incidentally, herein a "radio frequency device" refers to a device that is capable of reading data from and/or writing data to a radio frequency tag (e.g., an RFID tag).

Next in the injection process 56 of FIG. 3, the fluid 24 may be injected into a patient, as depicted by block 82. The fluid 24 may be injected by a power injector and syringe, intravenous drip, or manually operated syringe, for example. In some embodiments, the statistic or data corresponding to the statistic may be read by medical technician viewing the label on the container 20 prior to injecting the fluid 24. In some embodiments, the inclusion detector 10 may be communicatively interconnected with a medical fluid administration device (e.g., power injector, infusion pump, etc.). In such embodiments, the inclusion detector 10 may send a signal indicative of the calculated amount of inclusions in the medical fluid to the medical fluid administration device. For instance, if the medical fluid 24 includes more inclusions than what is determined to be acceptable, the inclusion detector 10 may send a signal to the medical fluid administration device preventing the administration device from being utilized to administer the fluid from the container to a patient. In the case that the inclusion detector 10 includes a radio frequency device and the container has a radio frequency tag associated therewith, the inclusion detector 10 can be utilized to write appropriate inclusion data to the tag of the container. In such a case, a medical fluid administration device having a radio frequency device associated therewith may read the inclusion data from the radio frequency tag and may utilize that data in determining whether or not to perform or allow performance of a medical fluid administration procedure utilizing that particular container.

Finally, the patient may be imaged, as depicted by block 84 of FIG. 3. Imaging the patient may include imaging the patient with a magnetic resonance imaging system, ultrasound system, fluoroscopy system, tomography system (e.g., a positron emission tomography scanning system), and/or projection radiography system (e.g., an x-ray system), for example.

In some embodiments, the imaging system utilized to image the patient may be communicatively interconnected with the inclusion detector 10. As such, the image data collected using the inclusion detector 10 (and/or other data relating to the container 20 and/or the medical fluid 24 disposed therein) may be transmitted to the imaging system. Likewise, data from the imaging system may be transmitted to the inclusion detector 10.

In some embodiments, the inclusion detector 10 may be communicatively interconnected with a hospital information system (HIS), which herein refers to any appropriate computerized healthcare facility management system. In such embodiments, inclusion data determined using the inclusion detector 10 may be transmitted to the hospital information system to track such data. In the case that the inclusion detector 10 includes a radio frequency device or bar code reading device, and the container has a corresponding radio frequency tag or bar-coded tag associated therewith, the inclusion detector 10 can be utilized to keep track of inventory that discarded due to an inappropriate amount of inclusions. In particular, the inclusion detector 10 can be utilized to associate medical fluid inclusion data with a bar code or radio frequency tag of the particular container housing the medical fluid.

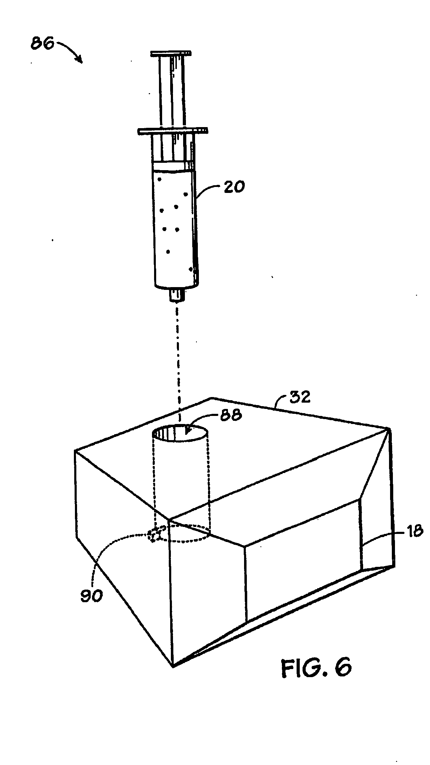

FIG. 6 illustrates an exemplary integrated bubble detector 86. As used herein, the term "integrated" indicates that components are joined together in such a manner that they can be separated from a larger assembly without separating the components from each other. In the embodiment of FIG. 6, the light shield 32 is coupled to the camera 18. A receptacle 88 receives the container 20, and a switch or container sensor 90 detects the presence of the container 20 in the receptacle 88. In some embodiments, the integrated bubble detector 86 may signal the camera 18 to capture an image in response to a signal from the container sensor 90 indicating the presence of a container 18.

In some embodiments, all or part of the inclusion detector 10 may be part of an injector. For example, the inclusion detector 10 may be integrated with a powered injector. Prior to, or during, injection, the inclusion detector 10 may capture an image of a syringe or a fluid in a tube leaving the syringe, analyze the image, and transmit a signal that indicates the quantity or presence of inclusions to a controller in the powered injector. In response, the controller in the powered injector may prevent an injection, stop an injection, or output a signal, such as an alarm. In another example, the inclusion detector 10 may be integrated into a manually operated injector. The inclusion detector 10 and manually operated injector may be configured to prevent operation of the manually operated injector upon a determination that a number or quantity of inclusions is greater than a threshold value. For instance, the alarm 16 of the inclusion detector 10 may include a solenoid configured to engage a latch or other appropriate device to obstruct the movement of a plunger in the syringe.

While the invention may be susceptible to various modifications and alternative forms, specific embodiments have been shown by way of example in the figures and have been described in detail herein. However, it should be understood that the invention is not intended to be limited to the particular forms disclosed. Rather, the invention is to cover all modifications, equivalents, and alternatives falling within the spirit and scope of the invention as defined by the following appended claims.

## Claims

1. A method of operation for a medical fluid injection system, the method comprising:
receiving a syringe containing a medical fluid;
generating a signal indicative of a quantity of image data indicative of inclusions in the medical fluid; and
determining whether the signal indicates that the quantity of inclusions exceeds a predetermined threshold value.

2. The method of claim 1, wherein the receiving comprises placing the syringe within a field of view of an image capture device.

3. The method of claim 1 or claim 2, further comprising injecting the medical fluid into the patient if the quantity of inclusions does not exceed the predetermined threshold value.

4. The method of claim 3, further comprising imaging the patient with a magnetic resonance imaging system, an ultrasound system, a fluoroscopy system, a tomography system, a projection radiography system, or a combination thereof.

5. The method of claim 1 or claim 2, further comprising preventing injection of the medical fluid into the patient in response to a determination that the quantity of inclusions in the medical fluid exceeds the predetermined threshold value.

6. The method of any preceding claim, wherein the quantity is determined by counting a number of pixels in at least a portion of the image.

7. The method of claim 6, wherein the counting comprises:
counting a number of pixels in at least one of a first row and a first column that include data indicative of at least a portion of an inclusion; and, then
counting a number of pixels in at least one of a second row and a second volume that include data indicative of at least a portion of an inclusion.

8. The method of claim 6 or claim 7, wherein the counting comprises calculating a percentage of pixels in an image that include data indicative of inclusions in the medical fluid.

9. The method of any of claims 6-8, wherein the counting comprises counting a number of pixels that include data indicative of a range of colour.

10. The method of any of claims 6-9, comprising calibrating an image based on data in the image that is representative of calibration marks.

11. An inclusion sensor for sensing inclusions in a syringe containing a medical fluid, the sensor comprising:
an image capture device;
an image processor communicatively interconnected with the image capture device; and
an inclusion identifier communicatively interconnected with the image processor and configured to quantify a number of inclusions indicated by the data in the image.

12. The sensor of claim 11, wherein the image capture device comprises a digital camera having a resolution of more than 100 pixels.

13. The sensor of claim 11 or claim 12, wherein the image processor comprises a microprocessor and dynamic random access memory.

14. The sensor claim of any of claims 11-13, further comprising an alarm communicatively interconnected with the image processor.

15. The sensor of any of claims 11-14, wherein the image capture device and a mount for a syringe are integrated with each other, and wherein the image capture device is aligned with the said mount.
